# EUROPEAN PATENT APPLICATION

(11) **EP 2 133 309 A1**
(43) Date of publication of application: **16.12.2009**
(21) Application number: 08736732.2
(22) Date of filing: 28.02.2008
(51) Int. Cl.: C01B 39/44, B01J 29/65, C07C 5/22

(54) **METHOD FOR PREPARATION OF AN ALUMINOSILICATE WITH FERRIERITE STRUCTURE FROM GELS CONTAINING TETRAMETHYL AMMONIUM AND BENZYLMETHYLPYRROLIDINE, AND USES THEREOF**

(30) Priority: 23.03.2007 ES 200700782
(71) Applicant: Consejo Superior De Investigaciones Científicas, 28006 Madrid (ES)
(72) Inventor: PINAR PRIETO, Ana Belén, E-28006 Madrid (ES); PÉREZ PARIENTE, Joaquín, E-28006 Madrid (ES); GÓMEZ-HORTIGÜELA SAINZ, Luis, E-28006 Madrid (ES)
(74) Representative: Pons Ariño, Angel
(86) International application number: PCT/ES2008/070035
(87) International publication number: WO 2008/116958

(57) **Abstract**

The present invention comprises a new methiod for hydrothermal synthesis of ferrierite zeolite, using tetramethyl ammounium and benzylmethylpyrrodlidine as directional structure agents, and does not require a subsequent process in order to obtain the acid form of the material. This aspect is very important as it avoids subject treatments of calcination and successive interchanges which degrade the structure of the zeolite. The synthetic ferrierite obtained by means of the invention process has uses in catalytic processes of transformation of hydrocarbons, such as cracking, isomerization, alkylation and polymerization. Specifically, it can be used in the isomerization of butene to isobutene. To be used in catalytic processes the zeolite ferrierite must be heated in an oxidant atmosphere in conditions such that the organic material occluded in the interior is eliminated.

## Description

### TECHNICAL FIELD OF THE INVENTION

This invention relates to a new method for preparing zeolite ferrierite, using tetramethylammonium and benzyl-methylpyrrolidinium as structure-directing agents, as well as the use thereof in hydrocarbon conversion reactions. Therefore, this invention relates to the preparation of zeolite materials and its application is framed within the chemical industry, more specifically, the Petrochemical industry.

### STATE OF THE ART

Ferrierite is a crystalline aluminosilicate that belongs to the group of tectosilicates called zeolites. This zeolite is found in nature as a mineral. The structure of ferrierite is characterised by having a system of channels parallel to the crystal's c-axis direction, delimited by rings formed by 10 tetrahedra, with a size of 4.2 x 5.4 Å. These channels are connected, by means of rings composed of 8 tetrahedra (size: 3.5 x 4.8 Å), to small polyhedric cavities called ferrierite-type cavities ([5⁸6⁶8²]), the inner diameter whereof is 6 x 7 Å.

The laboratory synthesis of this zeolite was disclosed, for the first time, by R. M. Barrer and D. T. Marshall (J. Chem. Soc., 484-497 (1964)), who used a method for hydrothermal crystallisation of aqueous gels containing an alkali metal hydroxide, aluminum hydroxide and silica, heated to a temperature between 340ºC and 400ºC.

Since then, different methods of obtaining ferrierite using various organic compounds that contain nitrogen, which act as "templates" during the zeolite material crystallisation process, have been published. Patent US No. 4,251,499 (Feb. 17 1981) claims the synthesis of ferrierite in the presence of piperidine and alkyl-substituted piperidines, and the sodium cation. Patent US Pat No. 5,985,238 (Nov. 16 1999) discloses the hydrothermal synthesis of ferrierite at alkaline pH from gels containing alkali metals and pyridine. All these preparation methods use organic molecules that contain one nitrogen atom and a no. of carbon atoms not greater than 6, in the presence of alkaline cations and at alkaline pH.

Ferrierite is used as a catalyst in olefin isomerisation reactions (EP 501,577; EP 523,838); to this end, it must contain acid centres. The generation of these acid centres requires calcination of the material in order to eliminate the organic compounds that are occluded in the interior thereof at the end of the synthesis, the exchange of the alkaline cations present in the material with ammonium ions, and subsequent calcination of the latter to generate the catalytically-active acid centres. Therefore, the methods of preparing ferrierite disclosed thus far require a subsequent treatment to generate acid centres in the material. In this process of calcination and successive exchanges, the structure of zeolite is degraded, and part of the aluminum atoms present therein are removed, which results in a decrease in the material's catalytic activity.

This invention discloses a new method for preparing ferrierite, using the organic compounds tetramethylammonium and benzyl-methylpyrrolidinium as structure-directing agents, which makes it possible to obtain the material with acid centres without the need for a subsequent treatment that includes calcination steps.

### DESCRIPTION OF THE INVENTION

### BRIEF DESCRIPTION

A first object of the invention is the method for preparing zeolite ferrierite, hereinafter method of the invention, by the hydrothermal crystallisation of aqueous gels, characterised by the use of tetramethylammonium and benzyl-methylpyrrolidinium as structure-directing agents.

A particular object of the invention is the method for preparing zeolite ferrierite **characterised in that** it comprises three steps:
(i) Preparation of a synthesis mixture,
(ii) thermal treatment of the synthesis mixture and
(iii) separation of the ferrierite crystals,
   such that synthesis mixture (i) comprises, in addition to the compounds tetramethylammonium and benzyl-methylpyrrolidinium, silicon and aluminum compounds, and fluoride ions.

A second object of the invention is the Zeolite Ferrierite obtained from the method of the invention.

A third object of the invention is the use of the zeolite ferrierite obtained from the method of the invention in catalytic hydrocarbon conversion processes, such as cracking, isomerisation, alkylation and polymerisation.

### DESCRIPTION OF THE INVENTION

This invention consists of a new method for the hydrothermal synthesis of zeolite ferrierite, using the organic compounds tetramethylammonium and benzyl-methylpyrrolidinium as structure-directing agents, which does not require a subequent process to obtain the acid form of the material. This fact is of great relevance, since it avoids subsequent treatments with calcination and successive exchanges, which degrade the structure of zeolite by removing part of the aluminum atoms present therein and cause a decrease in the material's catalytic activity. Furthermore, this method is **characterised in that** it is performed from gels that contain fluoride ions, in the absence of inorganic cations.

Therefore, one object of this invention is the method for preparing zeolite ferrierite, hereinafter method of the invention, by the hydrothermal crystallisation of aqueous gels, characterised by the use of the organic compounds tetramethylammonium and benzyl-methylpyrrolidinium as structure-directing agents.

Structure-directing agents are defined as those compounds which act as templates during the zeolite material crystallisation process. An essential characteristic of this method is the fact that these two structure-directing agents have different sizes, which affects their function during the method of the invention. Thus, tetramethylammonium has a small size and, therefore, fits inside the ferrierite-type cavity, which has a size of about 6 Å, whereas the size of benzyl-methylpyrrolidinium does not allow it to fit inside said cavity, but is sufficiently small to lodge inside the 10-tetrahedra channel. This method is also **characterised in that** the hydrothermal synthesis of the zeolite ferrierite crystals does not require the addition of metal, alkali or earth-alkaline, cations, although, optionally, they may be present in the synthesis gel, and in that the synthesis gel contains fluoride ions.

A particular object of the invention is the method of the invention wherein tetramethylammonium is in the form of the corresponding hydroxide, or mixtures of tetramethylammonium hydroxide and one or more tetramethylammonium salts, such as chloride, bromide or iodide.

Another particular object of the invention is the method of the invention wherein benzyl-methylpyrrolidinium is in the form of the corresponding hydroxide, or mixtures of hydroxide and salts such as chloride, bromide or iodide.

The organic additive benzyl-methylpyrrolidinium may be prepared in three steps. The first step comprises alkylation of the secondary amine pyrrolidine with a benzyl halide, whereof benzyl chloride is a non-limiting example, to obtain benzylpyrrolidine. In the second step, the benzyl-methylpyrrolidinium halide, such as, for example, iodide, is prepared by means of a reaction between the benzylpyrrolidine synthesised in the first step and the methyl iodide. In the third step, the benzyl-methylpyrrolidinium iodide may be totally or partially converted into the corresponding hydroxide by means of an ion exchange process, through contact between a benzyl-methylpyrrolidinium iodide solution and an ion exchange resin.

Therefore, a particular object of the invention is the method of the invention wherein the organic additive benzyl-methylpyrrolidinium is previously prepared by a process that comprises three steps:
- alkylation of the secondary amine pyrrolidine with a benzyl halide to produce benzylpyrrolidine
- preparation of the benzyl-methylpyrrolidinium halide by means of the reaction between benzylpyrrolidine and methyl halide
- ion exchange between the benzyl-methylpyrrolidinium halide and the corresponding hydroxide using an ion exchange resin.

Another particular object of the invention is the method of the invention, which comprises three steps:
(i) Preparation of a synthesis mixture,
(ii) thermal treatment of the synthesis mixture and
(iii) separation of the ferrierite crystals,
   hereinafter called particular method of the invention, wherein synthesis mixture (i) comprises, in addition to the organic compounds tetramethylammonium and benzyl-methylpyrrolidinium, silicon and aluminum compounds, and fluoride ions.

A particular embodiment of the invention is the particular method of the invention wherein the fluoride ions come from hydrofluoric acid.

A particular embodiment of the invention is the particular method of the invention wherein the silicon compound of synthesis mixture (i) is an alkoxide, such as tetramethoxysilane and tetraethoxysilane, a colloidal silica, or an amorphous or crystalline silica.

Another particular embodiment of the invention is the particular method of the invention wherein the aluminum compound of synthesis mixture (i) is an alkoxide, such as aluminum isopropoxide, or an aluminum salt, such as aluminum nitrate or sulfate, or a hydroxide.

Another particular embodiment of the invention is the particular method of the invention wherein synthesis mixture (i) comprises the structure-directing agents tetramethylammonium (TMA) and benzyl-methylpyrrolidinium (BMP), SiO₂, Al₂O₃ and fluoride ions (F), and its chemical composition is between the following ranges expressed in moles:
SiO₂/Al₂O₃ : 10-70
(TMA + BMP) / SiO₂ = 0.30-1.0
TMA/SiO₂ = 0.005-0.10
BMP / SiO₂ = 0.295-0.90
H₂O/SiO₂ = 4-50
F/SiO₂ = 0.20-1.5

The synthesis mixture may be prepared in the following manner: once the solution containing the two structure-directing agents is prepared, the precursor of the silicon compound is added thereto, at a temperature of between 5ºC and 80ºC, and kept under stirring at the specified temperature for a period of time between 1 minute and 24 hours. Once the silicon compound is added to the solution of the structure-directing agents, the aluminum compound is added and the resulting mixture is kept under stirring at the specified temperatures and for the specified periods of time. Subsequently, the source of fluoride ions, preferably hydrofluoric acid, is added. The resulting mixture is stirred at a temperature of between 5ºC and 80ºC, for a period of time between 1 minute and 24 hours.

Another particular embodiment of the invention is the particular method of the invention wherein the thermal treatment of synthesis mixture (ii) is performed at between 100ºC and 200ºC, and, preferably, between 120ºC and 180ºC.

Another particular embodiment of the invention is the particular method of the invention wherein the thermal treatment of synthesis mixture (ii) is performed for a period of time between 24 and 720 hours, and, preferably, between 72 and 360 hours.

The separation of the ferrierite crystals described in (iii) may be performed by filtering the solid product obtained following the thermal treatment of the synthesis mixture and, subsequently, washing with deionised water and drying.

Another object of the invention is the zeolite ferrierite, hereinafter ferrierite of the invention, obtained by means of the method of the invention.

The synthetic ferrierite obtained by the method of the invention has applications in catalytic processes, such as, for example, the isomerisation of butene to isobutene, which require the activation of ferrierite. To do so, it is necessary to heat the zeolite ferrierite obtained by the method described in this invention under such conditions that the organic matter occluded inside the crystals during the synthesis process is eliminated. The activation of zeolite is performed by exposing the latter, under high-temperature conditions, to an oxidising atmosphere, which may contain oxygen, such as, for example, air or ozone, at a temperature of between 150ºC and 600ºC.

The calcined material obtained by the method described in this invention has a chemical composition in the anhydrous state expressed by the following formula: Al₂O₃ : mSiO₂
where the value of m is between 10 and 80, preferably between 20 and 60.

The ferrierite of the invention is active and selective in catalytic hydrocarbon conversion processes, such as the isomerisation of olefins and, in particular, the isomerisation reaction from butene to isobutene.

Thus, another object of the invention is the use of the ferrierite of the invention in catalytic hydrocarbon conversion processes, such as cracking, isomerisation, alkylation and polymerisation.

Another particular object of the invention is the use of the ferrierite of the invention in the isomerisation of olefins.

Another particular embodiment of the invention is the use of the ferrierite of the invention in the isomerisation of butene to isobutene.

The isomerisation of butene to isobutene may be performed at a temperature that generally ranges between 200ºC and 500ºC, at a pressure between 1 atm and 10 atm. The isobutene selectivity depends on the specific reaction conditions, such as temperature, partial pressure of butene and reaction time. The materials obtained in this invention have been assayed as butene isomerisation catalysts under severe de-activation conditions, under the following reaction conditions: T = 400ºC; P = 1 atm; nitrogen/1-butene molar ratio = 4; WHSV = 25, using 0.1 grams of catalyst. Under these conditions, the materials obtained in this invention lead to an isobutene selectivity greater than 85% for a butene conversion of 10%, whereas, for a 30% conversion, the selectivity is greater than 50%. These selectivity results are very high, taking into consideration the severe reaction conditions, and give an idea of the potential that these catalysts have in olefin isomerisation reactions.

### DESCRIPTION OF THE FIGURES

Figure 1. Diffractogram (CuK□ radiation) of the ferrierite obtained following the method described in example 1. The reflection marked with an * corresponds to traces of quartz.
Figure 2. Diffractogram (CuK□ radiation) of the ferrierite obtained following the method described in example 2.
Figure 3. Isobutene selectivity of the ferrierites obtained following the methods described in examples 1 and 2, as compared to the selectivity of a sample of ferrierite obtained following the method described in the patent referred to in example 3, assayed under the reaction conditions described in example 3.

### EMBODIMENT EXAMPLES

The following examples illustrate the invention, but are not intended to limit the scope of the invention.

### Example 1. Preparation of ferrierite with an Si/Al ratio in the synthesis gel equal to 15.7

This example illustrates the preparation, following this invention, of ferrierite with an Si/Al ratio in the synthesis gel equal to 15.7. Firstly, we describe the synthesis of the benzyl-methylpyrrolidinium cation, which takes place in two steps. In the first step, the tertiary amine benzylpyrrolidine is synthesised, the alkylation whereof, in this case with methyl iodide, leads to the benzyl-methylpyrrolidinium halide.

### Synthesis of the benzyl-methylpyrrolidinium cation

### i) Synthesis of the tertiary amine benzylpyrrolidine

The synthesis method for the preparation of the tertiary amine benzylpyrrolidine comprises the alkylation of the secondary amine pyrrolidine with benzyl chloride. In order to prepare 73.0 g of benzylpyrrolidine, 78.2 g of benzyl chloride are first added, drop by drop, to a solution of 65.9 g of pyrrolidine (50% molar excess) in an organic solvent such as ethanol (200 ml). Subsequently, 128.1 g of potassium carbonate are added, in order to favour the alkylation reaction by displacing the equilibrium through the elimination of the hydrochloric acid. The molar composition of the reaction mixture is:
1.5 pyrrolidine : 1.0 benzyl chloride : 1.5 potassium carbonate

The reaction is performed in a round-bottom flask with a 1-litre capacity, which is kept under reflux, at 90ºC under magnetic stirring, for 48 h. The tertiary amine is filtered in order to eliminate the excess potassium carbonate, washing this solid with ethanol. The ethanol is eliminated by vacuum evaporation (60ºC) in a rotary evaporator. Optionally, the amine resulting from the reaction may be extracted with an organic solvent such as chloroform. In this case, it is necessary to subsequently eliminate the chloroform by vacuum heating. The potential water residues must be eliminated by the addition of a desiccant, such as potassium carbonate, which is subsequently filtered. The product obtained by any of the two methods is purified by vacuum distillation. Benzylpyrrolidine is the fraction that is distilled at a temperature of about 110ºC at an approximate pressure of 17 mmHg. It is obtained in the form of a colourless oil, with a yield of between 65% and 75%.

### ii) Synthesis of the salt of the benzyl-methylpyrrolidinium cation

The method for obtaining the salt of the benzyl-methylpyrrolidinium cation that will be used as the structure-directing agent in the synthesis of zeolites consists of two steps. In the first step, benzyl-methylpyrrolidinium iodide is prepared from the previously obtained benzylpyrrolidine. In the synthesis of zeolites described, the benzyl-methylpyrrolidinium cation may be added to the synthesis gel in its hydroxide form; for this reason, in a second step, the benzyl-methylpyrrolidinium iodide is subjected to an ion exchange process wherein it is totally or partially converted into benzyl-methylpyrrolidinium hydroxide.

Alkylation of the previously synthesised tertiary amine benzylpyrrolidine is performed by the addition, under cold conditions, of said amine (73.0 g) to a solution of 92.9 g of methyl iodide (50% molar excess) in 200 ml of ethanol. The reaction is performed in a round-bottom flask, which, during the first few minutes of the reaction, is introduced in an ice bath. The flask is kept capped, at ambient temperature and under magnetic magnetic stirring, for a period of not less than three days. During this time, the flask must be covered with aluminum foil in order to prevent the photodegradation of the quaternary ammonium salt product. Subsequently, the ethanol is eliminated by vacuum evaporation at a temperature of 60ºC with the help of a rotary evaporator. The resulting solid, orange in colour, is benzyl-methylpyrrolidinium iodide. It is purified by exhaustively washing with diethyl ether until the wash waters are colourless. The solid, which is now yellow in colour, is left to dry at ambient temperature, in order to allow for evaporation of the ether. In this manner, 99.4 g of benzyl-methylpyrrolidinium iodide are obtained (approximate yield of 72%).

Finally, the iodide may be totally or partially converted into the corresponding hydroxide by ion exchange. To this end, a 2 M aqueous solution of the benzyl-methylpyrrolidinium iodide obtained (99.4 g) is prepared and placed in a PE/PP bottle, jointly with 328 g of ion exchange resin. The solution is kept under stirring at ambient temperature for a period of not less than 3 days. Once this time has elapsed, the resin is eliminated by filtration. The resulting liquid, benzyl-methylpyrrolidinium hydroxide, is assayed with hydrochloric acid in order to determine its concentration. In this case, the quantity of product obtained is 50.7 g (80% yield). The synthesis of the zeolite materials described herein is performed with a very small quantity of water; therefore, it is necessary to concentrate the aqueous benzyl-methylpyrrolidinium hydroxide solution. To this end, part of the water is vacuum-evaporated in a rotary evaporator, at moderate temperature (not greater than 55ºC), in order to prevent the degradation of the organic compound. Using this method, in this case a 65% solution of benzyl-methylpyrrolidinium hydroxide by weight is obtained.

Subsequently, we describe the synthesis of the zeolite materials following this invention, using tetramethylammonium and benzyl-methylpyrrolidinium, the synthesis whereof has been previously described, as structure-directing agents.

### iii) Synthesis of the zeolite materials

The zeolite materials with a Si/Al ratio = 15.7 in the gel are obtained from synthesis gels with the following molar composition:
0.06 TMAOH : 0.48 BMP hydroxide : 0.48 HF : 0.031 Al₂O₃ : 0.969 SiO₂ : 4.6 H₂O
   where TMAOH refers to tetramethylammonium hydroxide.

The reaction is performed in a polypropylene glass with a 250-ml capacity. In the first place, an aqueous solution of the cations used as structure-directing agents is prepared. To this end, 5.28 g of tetramethylammonium hydroxide (25% aqueous solution by weight) are added to a solution that contains 33.36 g of benzyl-methylpyrrolidinium hydroxide (65% aqueous solution by weight). Subsequently, 48.32 g of tetraethoxysilane and 3.05 g of aluminum isopropoxide are added. The mixture is kept under stirring at ambient temperature until complete evaporation of both the alcohol formed in the hydrolysis of the Si and Al sources, and the excess water. Subsequently, 4.69 g of hydrofluoric acid (48% aqueous solution by weight) are added drop by drop. The resulting gel, which has a pH of about 10, is manually homogenised with the aid of a spatula or a mortar. Subsequently, the gel is introduced into steel autoclaves with teflon liners (20-ml capacity), which are subjected to the static hydrothermal treatment at 150ºC for selected periods of time. The solid products are filtered, washed with ethanol and water, and allowed to dry at ambient temperature overnight.

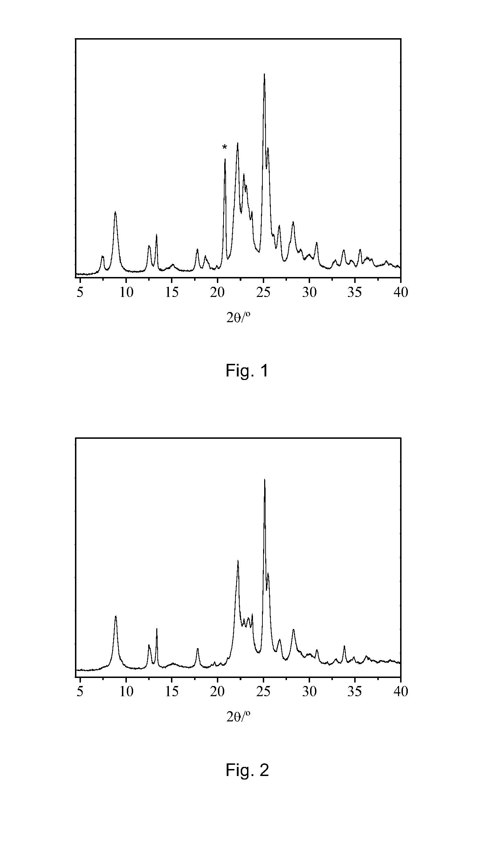

The diffractogram of the solid obtained is shown in figure 1, where the characteristic diffraction pattern of ferrierite may be observed. The solid's chemical composition is Si_{33.8}Al_{2.2}O₇₂. The dry solid product is subjected to calcination in an air atmosphere, and a 14% weight loss is observed, whereof at least 13.5% corresponds to organic matter and the rest corresponds to water.

### Example 2. Preparation of ferrierite with an Si/Al ratio in the synthesis gel equal to 11.

This example illustrates the preparation, following this invention, of ferrierite with an Si/Al ratio in the synthesis gel equal to 11. The synthesis of the benzyl-methylpyrrolidinium cation is performed following the method described in example 1.

This material is obtained from a synthesis gel with the following molar composition:
0.06 TMAOH : 0.48 BMP hydroxide : 0.453 HF : 0.043 Al₂O₃ : 0.957 SiO₂ : 4.6 H₂O
   where TMAOH refers to tetramethylammonium hydroxide.

The reaction is performed in a polypropylene glass with a 250-ml capacity. In the first place, an aqueous solution of the cations used as structure-directing agents is prepared. To this end, 5.10 g of tetramethylammonium hydroxide (25% aqueous solution by weight) are added to a solution that contains 33.26 g of benzyl-methylpyrrolidinium hydroxide (65% aqueous solution by weight). Subsequently, 47.37 g of tetraethoxysilane and 4.27 g of aluminum isopropoxide are added. The mixture is kept under stirring at ambient temperature until the complete evaporation of both the alcohol formed in the hydrolysis of the Si and Al sources, and the excess water. Subsequently, 4.41 g of hydrofluoric acid (48% aqueous solution by weight) are added drop by drop. The resulting gel, which has a pH of about 10, is manually homogenised with the aid of a spatula or a mortar. Subsequently, the gel is introduced into steel autoclaves with teflon liners (20-ml capacity), which are subjected to the static hydrothermal treatment at 150ºC for selected periods of time. The solid products are filtered, washed with ethanol and water, and allowed to dry at ambient temperature overnight.

The diffractogram of the solid obtained is shown in figure 2, where the characteristic diffraction pattern of ferrierite may be observed. The dry solid product is subjected to calcination in an air atmosphere, and a 15% weight loss is observed, whereof at least 14.5% corresponds to organic matter and the rest corresponds to water.

### Example 3. Evaluation of the catalytic activity of the ferrierites prepared following examples 1 and 2.

This example illustrates the behaviour of the samples obtained following the methods described in examples 1 and 2 as reaction catalysts in the isomerisation of butene to isobutene. For comparative purposes, a catalytic activity assay of a reference sample of zeolite ferrierite has been included, prepared following the method described in example 1 of patent US No. 4,016,245 (1977), which claims the preparation of a zeolite ferrierite from gels that contain sodium ions and the amine pyrrolidine.

In order to use the samples obtained following the method of the invention, it is necessary to activate the zeolite, which requires the elimination of the organic matter incorporated into the zeolite crystals during the synthesis process. To this end, both the samples obtained following this invention and the reference sample were activated by calcination under oxygen flow (4 litres/hour) enriched with ozone (3%-4%), at a temperature of 200ºC, for a period of time between 72 h and 120 h. Subsequently, the samples were heated under an air flow for 4 h at a temperature of 550ºC.

In the case of the reference sample, in order to exchange the sodium ions with protons and convert the material into an acid catalyst, after the treatment with an ozone flow and the subsequent calcination, the zeolite was twice exchanged with a 1 M ammonium chloride solution and, subsequently, filtered, washed, dried and calcined for 3 h at 350ºC and for another 3 h at 550ºC.

The reaction was performed in a fixed-bed reactor, under the following conditions: reaction temperature, 400ºC; pressure, 1 atmosphere; nitrogen/1-butene molar ratio = 4; WHSV = 25, using 0.1 grams of catalyst.

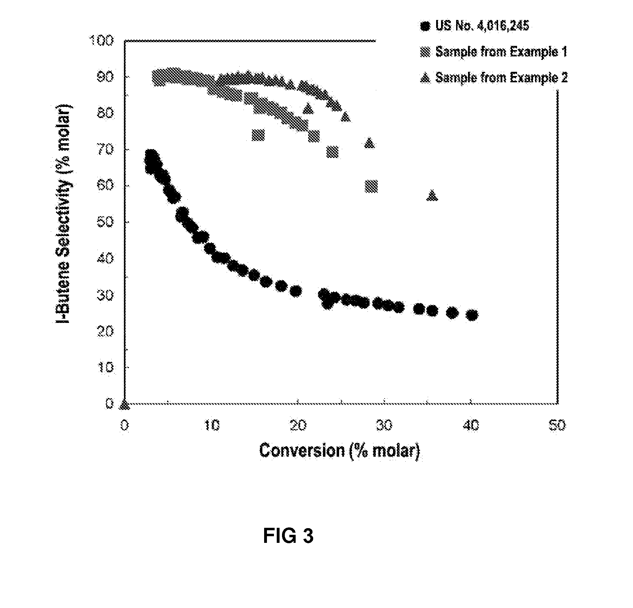

Figure 3 shows the isobutene selectivity of the three catalysts, and it is clearly observed that the two samples prepared as described in this invention are much more selective toward isobutene than the reference ferrierite sample.

The following table shows the three catalysts' selectivity toward the different reaction products:

| **Sample** | US No. 4,016,245 | | Example 1 | | Example 2 | |
|---|---|---|---|---|---|---|
| Conversion (% molar) | 9.84 | 30.48 | 10.27 | 28.58 | 11.01 | 28.29 |
| | Selectivity (% molar) | | | | | |
| Ethylene | 0.00 | 0.41 | 0.00 | 1.09 | 0.00 | 0.62 |
| Propane | 0.00 | 0.35 | 0.00 | 0.81 | 0.00 | 0.38 |
| Propylene | 18.99 | 26.85 | 1.63 | 16.61 | 1.61 | 8.57 |
| Iso-butane | 0.72 | 0.95 | 0.36 | 0.73 | 0.35 | 0.46 |
| N-butane | 1.29 | 1.26 | 0.80 | 4.27 | 0.63 | 2.77 |
| Iso-butene | 42.88 | 27.17 | 86.82 | 59.88 | 88.99 | 71.99 |
| Pentanes | 0.27 | 1.60 | 0.00 | 0.18 | 0.00 | 0.45 |
| Pentenes | 21.73 | 26.88 | 2.10 | 12.94 | 1.95 | 7.52 |
| Hexanes | 5.61 | 11.84 | 0.00 | 2.49 | 0.00 | 3.74 |
| Heptanes | 1.98 | 2.70 | 0.00 | 1.00 | 0.00 | 3.51 |
| C7⁺ | 6.54 | 0.00 | 8.29 | 0.00 | 6.48 | 0.00 |

This table shows that the two samples prepared following the method described in this invention have an isobutene selectivity that is at least twice that of the reference ferrierite. In fact, the samples prepared following examples 1 and 2 show an isobutene selectivity greater than 85% at a 10% conversion, whereas that of the reference zeolite does not exceed 43%. At a 30% conversion, the selectivity of the two samples of examples 1 and 2 is at least 60%, whereas that of the reference zeolite is less than 30%.

## Claims

1. Method for preparing zeolite ferrierite by means of hydrothermal crystallisation of aqueous gels, **characterised by** the use of tetramethylammonium and benzyl-methylpyrrolidinium as structure-directing agents.

2. Method for preparing zeolite ferrierite, as claimed in claim 1, **characterised in that** tetramethylammonium is in the form of the corresponding hydroxide, or as mixtures of tetramethylammonium hydroxide and one or more tetramethylammonium salts, such as chloride, bromide or iodide.

3. Method for preparing zeolite ferrierite, as claimed in claim 1, **characterised in that** benzyl-methylpyrrolidinium is in the form of the corresponding hydroxide, or as mixtures of the hydroxide and one or more salts, such as chloride, bromide or iodide.

4. Method for preparing zeolite ferrierite, as claimed in claim 1, **characterised in that** the organic additive benzyl-methylpyrrolidinium is previously prepared by means of a process that comprises three steps:
- alkylation of the secondary amine pyrrolidine with a benzyl halide to produce benzylpyrrolidine
- preparation of the benzyl-methylpyrrolidinium halide by means of the reaction between benzylpyrrolidine and methyl halide
- ion exchange of the benzyl-methylpyrrolidinium halide with the corresponding hydroxide using an ion exchange resin.

5. Method for preparing zeolite ferrierite, as claimed in claim 1, **characterised in that** it comprises three steps:
(i) Preparation of a synthesis mixture,
(ii) thermal treatment of the synthesis mixture and
(iii) separation of the ferrierite crystals,
such that synthesis mixture (i) comprises, in addition to the organic compounds tetramethylammonium and benzyl-methylpyrrolidinium, silicon and aluminum compounds, and fluoride ions.

6. Method for preparing zeolite ferrierite, as claimed in claim 5, **characterised in that** the fluoride ions come from hydrofluoric acid.

7. Method for preparing zeolite ferrierite, as claimed in claim 5, **characterised in that** the silicon compound of synthesis mixture (i) is an alkoxide, such as tetramethoxysilane and tetraethoxysilane, a colloidal silica, or an amorphous or crystalline silica.

8. Method for preparing zeolite ferrierite, as claimed in claim 5, **characterised in that** the aluminum compound of synthesis mixture (i) is an alkoxide, such as aluminum isopropoxide, an aluminum salt, such as aluminum nitrate and sulfate, or a hydroxide.

9. Method for preparing zeolite ferrierite, as claimed in claim 5, **characterised in that** synthesis mixture (i) comprises the organic compounds tetramethylammonium (TMA) and benzyl-methylpyrrolidinium (BMP), SiO₂, Al₂O₃ and fluoride ions (F), and the chemical composition thereof is between the following ranges expressed in moles:
SiO₂/Al₂O₃: 10-70
(TMA + BMP) / SiO₂ = 0.30-1.0
TMA / SiO₂ = 0.005-0.10
BMP / SiO₂ = 0.295-0.90
H₂O/SiO₂ = 4-50
F/SiO₂ = 0.20-1.5

10. Method for preparing zeolite ferrierite, as claimed in claim 5, **characterised in that** the thermal treatment of synthesis mixture (ii) is performed at between 100ºC and 200ºC, and, preferably, between 120ºC and 180ºC.

11. Method for preparing zeolite ferrierite, as claimed in claim 5, **characterised in that** the thermal treatment of synthesis mixture (ii) is performed for a period of time between 24 and 720 hours, and, preferably, between 72 and 360 hours.

12. Zeolite ferrierite obtained from the method described in claims 1-11.

13. Use of zeolite ferrierite obtained from the method described in claims 1-11 in catalytic hydrocarbon conversion processes, such as cracking, isomerisation, alkylation and polymerisation.

14. Use of zeolite ferrierite, as claimed in claim 13, in olefin isomerisation reactions.

15. Use of zeolite ferrierite, as claimed in claim 14, in isomerisation reactions from butene to isobutene.
